(19) Europäisches Patentamt

European Patent Office

Office européen des brevets

(11) EP 4 763 278 A1

## (12) EUROPEAN PATENT APPLICATION

(43) Date of publication:
24.06.2026 Bulletin 2026/26

(21) Application number: 25225128.5

(22) Date of filing: 18.12.2025

(51) International Patent Classification (IPC):
**A61N 5/10** (2006.01)

(52) Cooperative Patent Classification (CPC):
**A61N 5/1049;** A61N 2005/1059

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 18.12.2024 CN 202411875855

(71) Applicant: **Shanghai United Imaging Healthcare
Co., Ltd.
Shanghai 201807 (CN)**

(72) Inventors:
• **LI, Jiawei
Shanghai, 201807 (CN)**
• **LI, Chuanhao
Shanghai, 201807 (CN)**
• **LIAO, Can
Shanghai, 201807 (CN)**

(74) Representative: **Murgitroyd & Company
165-169 Scotland Street
Glasgow G5 8PL (GB)**

### (54) OBJECT POSITIONING METHOD AND RADIATION DELIVERY SYSTEM

(57) An object positioning method is provided, which includes obtaining a first posture image of a first posture of a target object in a first position, and determining first positions of one or more key points of the target object in the first posture image, obtaining a second posture image of a second posture of the target object in a second position after the first position, and determining second positions of one or more key points of the target object in the second posture image, and determining positioning information of the target object based on the first positions and the second positions.

## EP 4 763 278 A1

**Description**

**TECHNICAL FIELD**

The present disclosure relates to the field of medical technology, and in particular to an object positioning method, a radiation delivery system, an object positioning device, a radiation delivery apparatus, a storage medium, and a program product.

**BACKGROUND**

With the rapid development of medical equipment, users have increasingly high requirements for the accuracy of treatment using medical equipment. For example, when using radiotherapy equipment for inter-fraction treatment of patients, it is necessary for patients to be positioned multiple times for fractional treatments, so that the medical equipment can obtain the precise dose for the target area.

In the related art, radiotherapy positioning usually relies on laser lamps, and medical staff manually adjust the patient's position with the help of laser lines and body marking lines based on experience. This method heavily depends on the accuracy of the laser lamp placement, which places high requirements on installation and service engineers. Moreover, the repeatability of positioning in fractional treatments is limited by the skills and experience of medical staff, and there are deviations in fractional positioning performed by different medical staff, which restricts the use of radiotherapy equipment and prevents it from achieving optimal medical performance.

**SUMMARY**

In a first aspect of present disclosure, an object positioning method is provided, which includes: obtaining a first posture image of a first posture of a target object in a first position, and determining first positions of one or more key points of the target object in the first posture image; obtaining a second posture image of a second posture of the target object in a second position after the first position, and determining second positions of one or more key points of the target object in the second posture image; and determining positioning information of the target object based on the first positions and the second positions.

In some embodiments, the positioning information includes a positioning state and/or a positioning deviation of the second posture relative to the first posture.

In some embodiments, the object positioning method further includes determining whether the positioning state and/or the positioning deviation meet preset positioning requirements.

In some embodiments, the object positioning method further includes performing an identity authentication on the target object if the positioning state and/or the positioning deviation meet the preset positioning requirements.

In some embodiments, the object positioning method further includes obtaining a current posture image of the target object if the positioning state and/or the positioning deviation do not meet the preset positioning requirements, taking the current posture image as a new second posture image, determining second positions of one or more key points in the new second posture image, and determining the positioning information of the target object based on the first positions and second positions of the one or more key points in the new second posture image.

In some embodiments, the object positioning method further includes outputting the positioning information to the target object in a form of voice and/or image for guiding positioning of the target object.

In some embodiments, determining the first positions of the one or more key points of the target object in the first posture image includes identifying the one or more key points in the first posture image to obtain first position coordinate sets of the one or more key points in the first posture image, and determining the second positions of the one or more key points of the target object in the second posture image includes identifying the one or more key points in the second posture image to obtain second position coordinate sets of the one or more key points in the second posture image. The one or more key points in the first posture image are in one-to-one correspondence with the one or more key points in the second posture image. Determining the positioning information of the target object based on the first positions and the second positions includes determining the positioning information based on the first position coordinate sets and the second position coordinate sets.

In some embodiments, the positioning information includes a position deviation and an orientation deviation. Determining the positioning information based on the first position coordinate sets and the second position coordinate sets includes: performing a distance calculation on the first position coordinate sets and the corresponding second position coordinate sets to determine key point position deviations between the key points corresponding to the first position coordinate sets and the key points corresponding to the second position coordinate sets; performing an angle calculation on the first position coordinate sets and the corresponding second position coordinate set to determine key point orientation deviations between the key points corresponding to the first position coordinate sets and the key points corresponding to the second position coordinate sets; and determining the positioning information based on the key point position deviations and the key point orientation deviations.

In some embodiments, determining the positioning information based on the key point position deviations and the key point orientation deviations includes: performing a mean value calculation on all or part of the key point position deviations to determine an average position deviation of the second posture relative to the first posture; and performing a mean value calculation on all or part of the key point orientation deviations to determine an average orientation deviation of the second posture relative to the first posture.

In some embodiments, the positioning information includes a depth deviation. The object positioning method further includes: obtaining acquisition parameters for acquiring the second posture image and iris information of the target object; and determining the depth deviation of the second posture relative to the first posture based on the acquisition parameters and the iris information.

In some embodiments, the first position is an initial position of the target object during or before formulation of a current radiation delivery plan, and the second position is a delivery position of the target object after the formulation of the current radiation delivery plan and during or before the current radiation delivery.

In some embodiments, the object positioning method further includes moving the target object based on the positioning information to complete positioning of the target object.

In a second aspect of the present disclosure, a radiation delivery system is provided, which includes an image, a positioning device, and a support platform. The image acquisition device is configured to obtain a first posture image of a first posture of a target object in a first position on the support platform and a second posture image of a second posture of the target object in a second position on the support platform after the first position. The positioning device is configured to determine first positions of one or more key points of the target object in the first posture image and second positions of the one or more key points in the second posture image, and determine positioning information based on the first positions and the second positions.

In some embodiments, the support platform is configured to move the target object based on the positioning information to complete positioning of the target object.

In some embodiments, determining the first positions of the one or more key points of the target object in the first posture image and the second positions of the one or more key points in the second posture image includes: identifying the one or more key points in the first posture image to obtain first position coordinate sets of the one or more key points of the target object in the first posture image; and identifying the one or more key points in the second posture image to obtain second position coordinate sets of the one or more key points of the target object in the second posture image. The one or more key points in the first posture image are in one-to-one correspondence with the one or more key points in the second posture image. Determining the positioning information based on the first positions and the second positions includes determining the positioning information based on the first position coordinate sets and the second position coordinate sets.

Details of one or more embodiments of the present disclosure are set forth in the accompanying drawings and description below. Other features, objects, and advantages of the disclosure will become apparent from the description, the drawings, and the claims.

BRIEF DESCRIPTION OF THE DRAWINGS

To more clearly illustrate the technical solutions in the embodiments of the present disclosure or the related art, the following briefly introduces the drawings required for describing the embodiments of the present disclosure or the related art. Obviously, the drawings in the following description are only some embodiments of the present disclosure, and for those of ordinary skill in the art, other related drawings can be obtained according to these drawings without creative work.

FIG. 1 is a schematic structural diagram of a radiation delivery system according to some embodiments.
FIG. 2 is a schematic flowchart of a part of an object positioning method according to some embodiments.
FIG. 3 is a schematic flowchart of a part of an object positioning method according to some embodiments.
FIG. 4 is a schematic flowchart of a part of an object positioning method according to some embodiments.
FIG. 5 is a schematic flowchart of a part of an object positioning method according to some embodiments.
FIG. 6 is a schematic flowchart of a part of an object positioning method according to some embodiments.
FIG. 7 is a schematic flowchart of a part of an object positioning method according to some embodiments.
FIG. 8 is a schematic diagram of a positioning image according to some embodiments.
FIG. 9 is a schematic diagram of a display interface according to some embodiments.
FIG. 10 is a schematic flowchart of an object positioning method according to some embodiments.
FIG. 11 is a schematic structural diagram of a radiation delivery system according to some embodiments.
FIG. 12 is a structural block diagram of an object positioning device according to some embodiments.
FIG. 13 is an internal structural diagram of a radiation delivery apparatus according to some embodiments.

DETAILED DESCRIPTION

In order to make the purpose, technical solutions, and advantages of the present disclosure clearer, the present disclosure will be further described in detail below with reference to the accompanying drawings and embodiments. It should be understood that the specific embodiments described herein are only used to explain the present disclosure and are not used to limit the present disclosure.

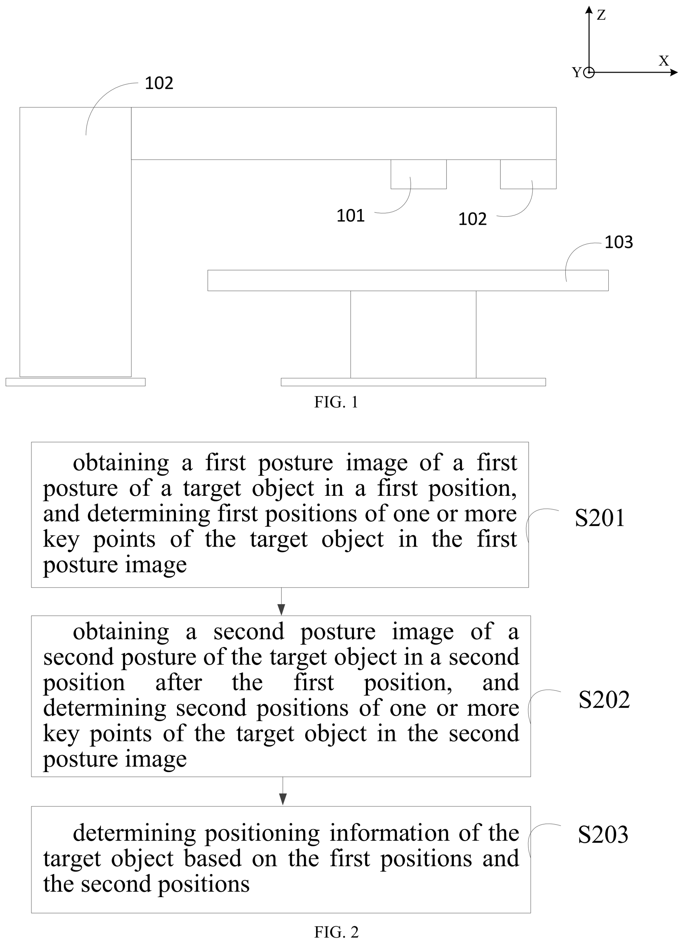

The positioning method provided in the embodiments of the present disclosure can be applied to the radiation delivery system shown in FIG. 1. The radiation delivery system includes an image acquisition device 101, an object positioning device 102, and a support platform 103. The object positioning device 102 is connected to the image acquisition device 101 and the support platform 103 in a wired or wireless manner. The image acquisition device 101 is configured to obtain posture images of a target object located on the support platform 103. The object positioning device 102 is configured to analyze the posture image obtained by the image acquisition device 101, thereby guiding the positioning of the target object on the support platform 103 based on the analysis result. The support platform 103 is configured to carry the target object and drive the target object to move for positioning. The image acquisition device 101 may be a camera, a video camera, or the like. The object positioning device 101 may store the image obtained by the image acquisition device 101. The object positioning device 101 may be, but is not limited to, various personal computers, notebook computers, smartphones, tablet computers, Internet of Things devices, projection devices, and the like. It should be noted that the positioning method provided in the embodiments of the present disclosure can be used for the above radiation delivery system, and can also be applied to other imaging systems or apparatuses with positioning requirements. The above radiation delivery system is only an illustration of an application requirement of the positioning method described in the embodiments of the present disclosure, and is not limited thereto.

Those skilled in the art can understand that the structure shown in FIG. 1 is only a block diagram of a part of the structure related to the solution of the present disclosure, and does not constitute a limitation on the radiation delivery system to which the solution of the present disclosure is applied. A specific radiation delivery system may include more or fewer components than those shown in the figure, or combine some components, or have different component arrangements. In this document, the terms "positioning" and "placement" can be used interchangeably.

In an exemplary embodiment, as shown in FIG. 2, an object positioning method is provided. Taking the method applied to the object positioning device in the radiation delivery system in FIG. 1 as an example, the method includes steps S201-S203.

In the step S201, a first posture image of a first posture of a target object in a first position is obtained, and first positions of one or more key points of the target object in the first posture image are determined.

In the step S202, a second posture image of a second posture of the target object in a second position after the first position is obtained, and second positions of one or more key points of the target object in the second posture image are determined.

In some embodiments, the first position may be an initial position of the target object during or before the formulation of a radiation delivery plan. Correspondingly, the first posture image may be a posture image of the target object acquired during positioning under the guidance of medical staff during or before the formulation of the radiation delivery plan, or a posture image of the target object acquired during positioning under the guidance of the radiation delivery system during or before the formulation of the radiation delivery plan. For example, the first posture image may be a posture image for an initial position, or a posture image for any of multiple fraction positions. That is, the first posture image may be a historical posture image during any treatment fraction. The second position is a delivery position after the formulation of the radiation delivery plan and during or before the radiation delivery. Correspondingly, the second posture image is a posture image of the target object during positioning after the formulation of the radiation delivery plan and during or before the radiation delivery. For example, the second posture image is the posture image of the target object in any of the first fraction, second fraction, third fraction, ..., Nth fraction after the first position. For instance, the second posture image can be the posture image during a current treatment fraction.

It can be understood that the acquisition of the first posture image and the acquisition of the second posture image may be at different time points. For example, a posture image corresponding to any previous treatment fraction is obtained as the first posture image. When it is necessary to perform positioning of the target object for a subsequent treatment fraction, a posture image corresponding to the current treatment fraction is obtained as the second posture image. In some embodiments, after the acquisition of the first posture image and the determination of the first positions of the one or more key points, the first posture image and the corresponding first positions may be stored in, for example, a memory for subsequent use. In other embodiments, the first posture image, the first positions of the one or more key points in the first posture image, the second posture image, and the second positions of the one or more key points in the second posture image are all pre-stored in the memory for subsequent use.

In the embodiments of the present disclosure, during or before the formulation of the radiation delivery plan, when a first position of the target object with a first posture is determined, the image acquisition device is activated to obtain a posture image of the first posture of the target object as the first posture image. For example, after the first position and after the formulation of the radiation delivery plan, during or before the radiation delivery, when needing to place the target object in a second position, the image acquisition device is controlled to obtain the current image of the target object that can reflect the posture of the target object, and the obtained image is used as the second posture image. Specifically, the above process of positioning and obtaining the posture image may be a cyclic process. For example, when the target object in placed in a first position, the image acquisition device is activated to obtain a posture image of the target object as an initial first posture image. When the target object is placed in a second position for the first time after the first position, the image acquisition device is activated to obtain a posture image of the target object as the second posture image for a first fraction. When the target object is placed in a second position for the second time after the second position for the first time, the image acquisition device is activated to obtain a posture image of the target object as a second posture image for a second fraction. When the target object is placed in a second position for the third time after the second position for the second time, the image acquisition device is activated to obtain a posture image of the target object as a second posture image for a third fraction. This cycle continues until the last second posture image is obtained. It should be noted that when the first posture image is obtained in the first position, the first posture image may be saved for use when analyzing the positioning information in the next fraction. The positioning information may include various types of information associated with positioning. As a non-limiting example, the positioning information may include the posture or state of the target object, positioning error, recommended positioning direction, recommended positioning distance, whether the positioning result or process meets a specified standard, whether positioning is being performed, or the like. In some embodiments, when the second posture image is obtained in each second position, all the second posture images of the second positions may be saved for the user to view at any time.

The key points may include reference points (e.g., body points or joint points, etc.) strongly related to the posture, motion state, identity, etc., of the target object, or the key points are reference points that are important or indispensable for determining the posture, motion state, identity, etc., of the target object. Taking the target object as a human being as an example, the key points may include body key points and facial key points, where the body key points may include joint points on the body, points on the body's axis of symmetry, and the like. The facial key points may include feature points on the facial features, points on the facial axis of symmetry, and the like. Taking the target object as an object as an example, the key points may include a center point, a vertex, and the like. The key points may include position points on the surface of the object, or position points inside the object that can be predicted or inferred based on the points on the surface of the object.

In the embodiments of the present disclosure, an optional implementation is that, when the object positioning device obtains the first posture image based on the foregoing steps, a position recognition may be performed on the one or more key points included in the first posture image, such that the position coordinate sets of the first positions of the key points are identified, and the identified position coordinate sets of the first positions of the key points are saved to a database. When the object positioning device obtains the second posture image of the next fraction based on the foregoing steps, a position recognition is performed on the one or more key points included in the second posture image, such that the position coordinate sets of the second positions of the key points are identified, the position coordinate sets of the first positions are extracted from the database, and the positioning information of the target object can be analyzed and obtained according to the position coordinate sets of the first positions and the position coordinate sets of the second positions. According to the above manner, every time the object positioning device acquires a posture image, it can recognize the positions of the key points in the posture image, and the identified position coordinate sets of the key points are then saved so that they can be called at any time when needed. According to another optional manner, when the object positioning device obtains the first posture image based on the foregoing steps, the first posture image is saved to the database. When the object positioning device obtains the second posture image of the next fraction based on the foregoing steps, the first posture image is extracted from the database, and a position recognition is performed on the key points in the first posture image and the second posture image respectively to obtain the position coordinate sets of the first positions of the key points in the first posture image and the position coordinate sets of the second positions of the key points in the second posture image. Then, the positioning information of the target object is analyzed and obtained according to the position coordinate sets of the first positions and the position coordinate sets of the second positions. In this manner, each time the object positioning device obtains a posture image, the posture image is first saved. When it is necessary to analyze the positioning information of different fractions, the posture images of different fractions are extracted from the database to perform position recognition of key points, so as to determine the positioning information of the target object for each second position relative to the first position.

In the step S203, positioning information of the target object based on the first positions and the second positions is determined.

The positioning information may include a positioning state and/or a positioning deviation of the second posture relative to the first posture of the target object. Optionally, the positioning information is used to guide the positioning of the target object. The positioning information is output to the target object in the form of voice and/or image for guiding the positioning of the target object. The positioning information may include a positioning direction for positioning the target object and a positioning distance for positioning the target object. The positioning direction may be expressed using conventional terms indicating directions such as "left", "right", "up", "down", "forward", "backward", and the like. The positioning information further includes a guide image, and the guide image includes an image or outline of the target object and a positioning identifier. The positioning identifier includes a direction identifier and a distance identifier. The direction identifier is used to instruct the positioning of the target object according to the direction of the direction identifier. The distance identifier is used to instruct the positioning of the target object according to the distance identifier.

In the embodiments of the present disclosure, when the object positioning device obtains the first positions of the one or more key points of the target object in the first posture image and the second positions of the one or more key points in the second posture image based on the foregoing steps, the position deviation between the first positions of each key point in the first posture image and the second positions of the corresponding each key point in the second posture image can be analyzed. Further, the positioning state and/or positioning deviation of the second posture relative to the first posture can be determined based on the position deviation corresponding to each key point. Consequently, positioning information including a positioning direction and a positioning distance, positioning information including a positioning image, or positioning information including a positioning direction, a positioning distance, and a positioning image is then generated based on the positioning state and/or positioning deviation of the second posture relative to the first posture, so as to guide the positioning of the target object.

The method described in the above embodiments is applied to a radiation delivery system. The radiation delivery system obtains a first posture image of a first posture of a target object in a first position and a second posture image of a second posture of the target object in a second position after the first position, determines first positions of one or more key points of the target object in the first posture image and second positions of the one or more key points in the second posture image, and determines positioning information of the target object based on the first positions and the second positions. The above method realizes a method for automatically guiding the positioning of a target object by using a radiation delivery system. Specifically, by real-time comparing the postures of the target object under different positions, the positioning state or positioning deviation of the target object between different fractions can be quickly found and corrected, so that the target object can adjust its posture by itself according to the prompt or guidance of the radiation delivery system, improving the positioning efficiency. Moreover, the above method determines the positioning information by identifying the positions of key points in different posture images, which can accurately determine the positioning deviation between different placement postures of the target object, thereby accurately performing positioning guidance, and solving the repeatability problem of the target object in multiple positionings to a certain extent.

In an exemplary embodiment, a method for determining positioning information is provided. The method includes determining a positioning state and/or a positioning deviation of the second posture relative to the first posture as the positioning information based on the first positions and the second positions.

The positioning state can be used to indicate whether the second posture of the target object is consistent with the first posture, or whether the second posture of the target object meets the requirements. The positioning deviation may include at least one of a distance deviation, an orientation deviation, and a depth deviation. The distance deviation refers to the distance deviations between the position of the target object in the second position on the three axes of the three-dimensional space (e.g., the X-axis, Y-axis, and Z-axis, where the directions of the three axes can be set arbitrarily as long as the three axes are perpendicular to each other) and the initial position of the target object in the first position on the three axes of the three-dimensional space. The orientation deviation refers to the deviation between the orientation of the target object in the second position and the orientation of the initial position of the target object in the first position. The depth deviation refers to the distance between the target object and the image acquisition device in the Z direction in the image. The Z direction is, for example, a direction perpendicular to the support platform 103.

In the embodiments of the present disclosure, when the object positioning device determines the first positions of the key points in the first posture image and the second positions of the key points in the second posture image, it can further determine the positioning information of the target object according to the first positions and the second positions. Specifically, it can analyze the positioning state of the second posture relative to the first posture, or analyze the positioning deviation of the second posture relative to the first posture, then use the above positioning state and/or positioning deviation as the positioning information. For example, through the difference between the first positions and the second positions, at least one of the distance deviation, orientation deviation, or depth deviation between the target object in the first position and in the second position is analyzed. And/or, it is analyzed whether the postures of the target object in the first position and in the second position are consistent, or whether the posture state corresponding to the target object in the second position meets the positioning requirements. Further, the object positioning device can also analyze the distance value that the target object needs to move according to the distance deviation in the positioning deviation, and analyze the specific direction that the target object needs to move according to the orientation deviation in the positioning deviation. Then, the object positioning device can convert the distance value that the target object needs to move and the specific direction that the target object needs to move into an expression for guiding movement, and perform positioning guidance based on the expression. Optionally, when the object positioning device determines the positioning information of the target object based on the foregoing steps, it can also generate a guide image according to the positioning state and/or positioning deviation. Following the above, when the positioning information includes a distance value and a direction, the positioning device can output the positioning information by means of voice broadcast or display, so that the target object can perform positioning according to the positioning information. When the positioning information includes a guide image, the positioning device can output the positioning information by means of display, so that the target object can clearly see the positioning identifier and perform positioning according to the positioning identifier.

In an exemplary embodiment, an implementation for identifying key points is provided. Determining the first positions of the one or more key points of the target object in the first posture image includes a step S301.

In the step S301, the one or more key points in the first posture image are identified to obtain a first position coordinate set of each of the key points.

The key points in the first posture image may be key points of all regions on the target object in the first posture image, such as key points of the whole body. Optionally, the key points in the first posture image may also be several key points of a specified region on the target object, such as key points of the upper body, key points of the face, or the like. The first position coordinate set includes an X-axis coordinate, a Y-axis coordinate, and a Z-axis coordinate in three-dimensional space.

In the embodiments of the present disclosure, the radiation delivery system can realize the identification of key points in the first posture image based on any type of neural network model to obtain the first position coordinate set of each key point. Specifically, the radiation delivery system can use a neural network model in an existing model library to identify key points in the image, or train a neural network model online based on sample data, and use the trained neural network model to identify key points in the first posture image to obtain the first position coordinate set of each key point. For example, the radiation delivery system uses MediaPipe built based on the BlazePose algorithm (which is a lightweight convolutional neural network architecture for real-time human posture estimation), and uses a lightweight CNN model that can be deployed on low-cost CPU devices to identify the positions of human key points. Optionally, when the key points are several key points of a specified region on the target object, the radiation delivery system needs to first determine the specified region in the first posture image, then identify the key points in the specified region to obtain the first position coordinate set of each key point. The above process can be realized by using two neural network models. For example, a segmentation model or classification model based on a neural network architecture is used to segment the image of the specified region in the first posture image, and then an identification model based on a neural network architecture is used to identify the image of the specified region to identify the first position coordinate set of each key point.

The step of determining the second positions of the one or more key points of the target object in the second posture image includes a step S302.

In the step S302, the one or more key points in the second posture image are identified to obtain a second position coordinate set of each of the key points.

The key points in the second posture image may be key points of all regions on the target object in the second posture image, such as key points of the whole body. Optionally, the key points in the second posture image may also be several key points of a specified region on the target object, such as key points of the upper body or key points of the face, or the like. If the key points in the first posture image and the key points in the second posture image are both several key points of a specified region, the number and type of the key points in the first posture image are consistent with those of the key points in the second posture image, that is, the key points in the first posture image are in one-to-one correspondence with the key points in the second posture image. For example, a key point in the first posture image and the corresponding key point in the second posture image point to the same part of the target object. The second position coordinate set includes an X-axis coordinate, a Y-axis coordinate, and a Z-axis coordinate in three-dimensional space.

In the embodiments of the present disclosure, the radiation delivery system can realize the identification of key points in the second posture image based on any type of neural network model to obtain the second position coordinate set of each key point. Specifically, the radiation delivery system can use a neural network model in an existing model library to identify key points in the image, or train a neural network model online based on sample data, and use the trained neural network model to identify key points in the second posture image to obtain the second position coordinate set of each key point. For example, the radiation delivery system uses MediaPipe built based on the BlazePose algorithm (which is a lightweight convolutional neural network architecture for real-time human posture estimation), and uses a lightweight CNN model that can be deployed on low-cost CPU devices to identify the positions of human key points. Optionally, when the key points are several key points of a specified region on the target object, the radiation delivery system needs to first determine the specified region in the second posture image, then identify the key points in the specified region to obtain the second position coordinate set of each key point. The above process can be realized by using two neural network models. For example, a segmentation model or classification model based on a neural network architecture is used to segment the image of the specified region in the second posture image, and then an identification model based on a neural network architecture is used to identify the image of the specified region to identify the second position coordinate set of each key point.

Following the above steps, when the object positioning device determines the first position coordinate sets of the key points in the first posture image and the second position coordinate sets of the key points in the second posture image, it can further determine the positioning information of the target object according to the first position coordinate sets and the second position coordinate sets. Specifically, since the position coordinate set of the key point can be X-axis, Y-axis, and Z-axis coordinates in the three-dimensional space, when performing calculations for the key points in the first posture image and the key points in the second posture image, the difference between the position coordinate sets of the key point in the first posture image and the key point in the second posture image on the X-axis, the difference between the position coordinate sets of the key point in the first posture image and the key point in the second posture image on the Y-axis, and the difference between the position coordinate sets of the key point in the first posture image and the key point in the second posture image on the Z-axis can be calculated respectively. Here, the position coordinate differences of all key points can be calculated simultaneously, or the position coordinate differences of all key points can be calculated sequentially. When all the position coordinate differences of the key points in the first posture image and the key points in the second posture image on the three axes are determined, for each axis, a mean value calculation can be performed on all position coordinate differences of the key points in the first posture image and the key points in the second posture image on each axis to obtain the positioning deviation of the second posture relative to the first posture on each axis.

For example, the first posture image includes three key points: A (x1, y1, z1), B (x2, y2, z2), C (x3, y3, z3), and the second posture image includes three key points: D (x4, y4, z4), E (x5, y5, z5), F (x6, y6, z6). The key point A corresponds to the key point D. For the key point A and the key point D, the position coordinate deviation on the X-axis is x4-x1, the position coordinate deviation on the Y-axis is y4-y1, and the position coordinate deviation on the Z-axis is z4-z1. The key point B corresponds to the key point E. For the key point B and the key point E, the position coordinate deviation on the X-axis is x5-x2, the position coordinate deviation on the Y-axis is y5-y2, and the position coordinate deviation on the Z-axis is z5-z2. The key point C corresponds to the key point F. For key point C and key point F, the position coordinate deviation on the X-axis is x6-x3, the position coordinate deviation on the Y-axis is y6-y3, and the position coordinate deviation on the Z-axis is z6-z3. Finally, the mean deviation of the position coordinate sets of all key points on the X-axis is calculated, that is ((x4 - x1) + (x5 - x2) + (x6 - x3)) / 3, the mean deviation of the position coordinate sets of all key points on the Y-axis is calculated, that is, ((y4 - y1) + (y5 - y2) + (y6 - y3)) / 3, and the mean deviation of the position coordinate sets of all key points on the Z-axis is calculated, that is, ((z4 - z1) + (z5 - z2) + (z6 - z3)) / 3. The mean deviations of the position coordinate sets on the three axes are then taken as the positioning deviations on the three axes.

The method described in the above embodiments can accurately determine the positioning deviation by identifying the key points in the first posture image and the second posture image to determine the positioning deviation, thereby accurately performing positioning guidance, solving the repeatability problem of the target object in multiple positionings to a certain extent, and improving the accuracy of the dose projection of the radiation delivery system in the later stage.

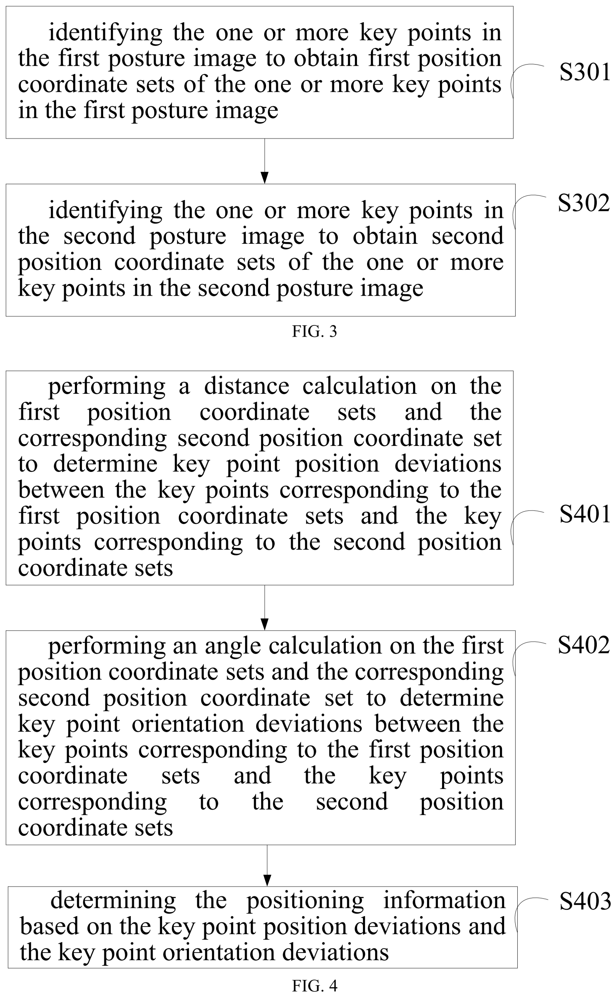

In an exemplary embodiment, when the positioning deviation includes a position deviation and an orientation deviation, another implementation for determining positioning information is provided. As shown in FIG. 4, the method for determining the positioning information based on the first position coordinate sets and the second position coordinate sets includes steps S401-S403.

In the step S401, a distance calculation is performed on each of the first position coordinate sets and the corresponding second position coordinate to determine a key point position deviation between the key point corresponding to each of the first position coordinate sets and the key point corresponding to each of the second position coordinate sets.

The key point position deviation essentially represents the distance deviation between two key points.

In the embodiments of the present disclosure, the radiation delivery system may use an Euclidean distance calculation method or other distance calculation methods to perform distance calculation on each of the first position coordinate sets and the corresponding each of the second position coordinate sets to obtain the distance deviation between the key point corresponding to each of the first position coordinate sets and the key point corresponding to each of the second position coordinate sets, and use the distance deviation as the key point position deviation. For example, the first posture image includes three key points: A (x1, y1, z1), B (x2, y2, z2), C (x3, y3, z3), and the second posture image includes three key points: D (x4, y4, z4), E (x5, y5, z5), F (x6, y6, z6). Then, the Euclidean distance D1 between the key point A and the key point D can be calculated using the following relational formula (1), the Euclidean distance D2 between the key point B and the key point E can be calculated using the following relational formula (2), and the Euclidean distance D3 between the key point C and the key point F can be calculated using the following relational formula (3):

$$D1 = \sqrt{(x1-x4)^2 + (y1-y4)^2 + (z1-z4)^2} \qquad (1);$$

$$D2 = \sqrt{(x2-x5)^2 + (y2-y5)^2 + (z2-z5)^2} \qquad (2);$$

$$D3 = \sqrt{(x3-x6)^2 + (y3-y6)^2 + (z3-z6)^2} \qquad (3).$$

In the step S402, an angle calculation is performed on each of the first position coordinate sets and the corresponding second position coordinate to determine a key point orientation deviation between the key point corresponding to each of the first position coordinate sets and the key point corresponding to each of the second position coordinate sets.

The key point orientation deviation essentially represents the orientation deviation between two key points, for example, indicating whether the directions of the two key points are consistent or opposite.

In the embodiments of the present disclosure, the radiation delivery system may use a cosine similarity calculation method or other similarity calculation methods to perform angle calculation on each of the first position coordinate sets and the corresponding second position coordinate to obtain the orientation deviation between the key point corresponding to each of the first position coordinate sets and the key point corresponding to each of the second position coordinate sets, and use the orientation deviation as the key point orientation deviation. For example, the first posture image includes three key points: A (x1, y1, z1), B (x2, y2, z2), C (x3, y3, z3), and the second posture image includes three key points: D (x4, y4, z4), E (x5, y5, z5), F (x6, y6, z6). Then, the orientation deviation F1 between the key point A and the key point D, the orientation deviation F2 between the key point B and the key point E, and the orientation deviation F3 between the key point C and the key point F can be calculated using the following relational formula group (4):

$$\cos\theta = \frac{(M \cdot N)}{(|M| \times |N|)}$$

$$M \cdot N = x_m \times x_n + y_m \times y_n + z_m \times z_n$$

$$|M| = \sqrt{x_m{}^2 + y_m{}^2 + z_m{}^2}$$

$$|N| = \sqrt{x_n{}^2 + y_n{}^2 + z_n{}^2} \qquad (4)$$

where $M(x_m, y_m, z_m)$ represents the position coordinate set of any key point in the first posture image, and $N(x_n, y_n, z_n)$ represents the position coordinate set of any key point in the second posture image.

In the step S403, the positioning information is determined based on the key point position deviations and the key point orientation deviations.

In the embodiments of the present disclosure, the positioning information may include the position deviation of each key point on the target object and the orientation deviation of each key point. When the radiation delivery system obtains all the key point position deviations based on the foregoing steps, it can use the key point position deviation of each key point as the position deviation in the positioning information. When the radiation delivery system obtains all the key point orientation deviations based on the foregoing steps, it can use the key point orientation deviation of each key point as the orientation deviation in the positioning deviation.

The method described in the above embodiments evaluates the deviation between the initial posture and the fraction posture of the target object from two dimensions of distance and direction, which can thus comprehensively evaluate the posture change of the target object, thereby accurately guiding the positioning of the target object and improving the positioning efficiency.

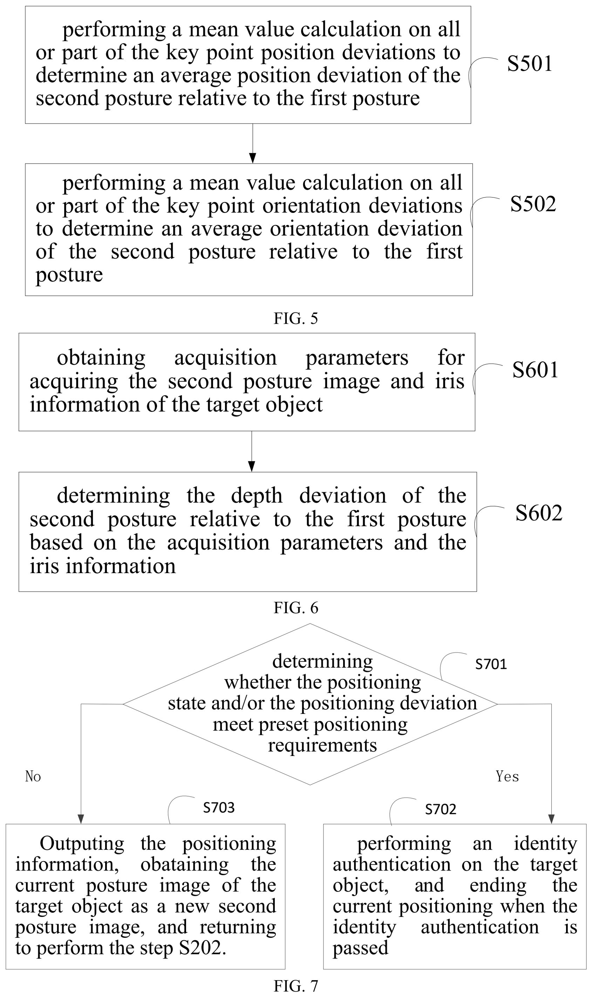

In an exemplary embodiment, an implementation for determining the positioning information based on the key point position deviations and the key point orientation deviations is provided, that is, the implementation of determining the positioning information based on the key point position deviations and the key point orientation deviations in the step 403. As shown in FIG. 5, the method includes steps S501-S502.

In the step S501, a mean value calculation is performed on all or part of the key point position deviations to determine an average position deviation of the second posture relative to the first posture.

In the embodiments of the present disclosure, when the radiation delivery system obtains the key point position deviations of all key points or part of the key points based on the foregoing steps, it can perform mean value calculation on the key point position deviations of all key points or part of the key points to obtain the average position deviation of the second posture relative to the first posture. For example, based on the example of the above embodiment in FIG. 4, the mean value calculation can be performed on the key point position deviations D1, D2, and D3 of the three key points, and the obtained distance mean value is taken as the position deviation of the second posture relative to the first posture.

In the step S502, a mean value calculation is performed on all or part of the key point orientation deviations to determine an average orientation deviation of the second posture relative to the first posture.

In the embodiments of the present disclosure, when the radiation delivery system obtains the key point orientation deviations of all key points or part of the key points based on the foregoing steps, it can perform mean value calculation on the key point orientation deviations of all key points or part of the key points to obtain the orientation deviation of the second posture relative to the first posture. For example, based on the example of the above embodiment in FIG. 4, the mean value calculation can be performed on the key point orientation deviations F1, F2, and F3 of the three key points, and the obtained orientation mean value is taken as the orientation deviation of the second posture relative to the first posture.

The method described in the above embodiment determines the position deviation of the second posture relative to the first posture by calculating the mean value of the key point position deviations of all or part of the key points, and determines the orientation deviation of the second posture relative to the first posture by calculating the mean value of the key point orientation deviations of all or part of the key points, which can realize the overall evaluation of the deviation between different postures of the target object from two dimensions of distance and direction, comprehensively evaluate the posture change of the target object, thereby accurately guiding the positioning of the target object and improving the positioning efficiency.

In an exemplary embodiment, the positioning information includes a depth deviation. Based on this, on the basis of the embodiment shown in FIG. 4, as shown in FIG. 6, the positioning method further includes steps S601-S602.

In the step S601, acquisition parameters for acquiring the second posture image and iris information of the target object are obtained.

The acquisition parameters include a field of view angle parameter and an image width. The iris information includes the actual size of the iris of the target object and the pixel size of the iris. The image acquisition device may be a device for acquiring images, such as a camera or a video camera.

In the embodiments of the present disclosure, when the radiation delivery system needs to calculate the depth deviation, the radiation delivery system may first obtain the configuration parameters of the image acquisition device used to capture the second posture image, and obtain the acquisition parameters including the field of view angle parameter and the image width from the configuration parameters. In practical applications, the object positioning device in the radiation delivery system may be communicatively connected to the image acquisition device. When the object positioning device needs to obtain the acquisition parameters, it can read the acquisition parameters of the image acquisition device, or the image acquisition device can also transmit the acquisition parameters to the object positioning device. Correspondingly, the radiation delivery system can perform iris recognition based on the first posture image or the second posture image of the target object to detect the actual size of the iris of the target object and the pixel size of the iris, so as to obtain the iris information of the target object. Optionally, the radiation delivery system can also obtain the iris information of the target object from a case database corresponding to the target object. Optionally, since the actual size and pixel size of the iris of different people are not much different, the iris information of the target object can be obtained from standard index data, for example, in general, the actual size of the iris is 11.7mm.

In the step S602, the depth deviation of the second posture relative to the first posture is determined based on the acquisition parameters and the iris information.

The embodiments of the present disclosure involve a specific calculation method of the depth deviation. When the radiation delivery system obtains the acquisition parameters of the image acquisition device and the iris information of the target object based on the foregoing steps, the depth distance corresponding to the first posture image and the depth distance corresponding to the second posture image can be calculated using the following relational formula group (5). Then, the difference between the two depth distances is calculated to obtain the depth deviation.

$$FOV_{rad} = FOV \times \left(\frac{\pi}{180}\right)$$

$$focal\_length\_px = \frac{width}{\left(2 \times \tan\left(\frac{FOV_{rad}}{2}\right)\right)}$$

$$dis\tan ce_{mm} = \frac{real\_iris\_size_{mm} \times focal\_length\_px}{iris\_diameter_{px}} \qquad (5)$$

where *FOV* represents the field of view angle parameter, $FOV_{rad}$ represents the field of view angle parameter after radian conversion, *width* represents the image width of the image captured by the image acquisition device, *focal_length_px* represents the focal length of the image acquisition device, *real_iris_size$_{mm}$* represents the actual size of the iris, *iris_diameter$_{px}$* represents the pixel size of the iris, and *distance$_{mm}$* represents the depth distance.

It can be understood that in some embodiments, the parameter information of the first posture image, such as the acquisition parameters, can be directly obtained from the storage, and the depth distance corresponding to the first posture image can be calculated based on the above formula group (5). In addition, the depth distance corresponding to the first posture image can be calculated and stored in advance for subsequent use.

The method described in the above embodiment also evaluates the deviation of the second posture relative to the first posture from the depth distance between the image acquisition device and the target object, which more comprehensively evaluates the posture change of the target object, thereby accurately guiding the positioning of the target object and improving the positioning efficiency.

In an exemplary embodiment, during each positioning process, if the positioning information includes a positioning state and/or a positioning deviation, the positioning state and/or the positioning deviation can also be evaluated to determine whether the positioning state and/or the positioning deviation are correct or meet the positioning requirements. Based on this, on the basis of the embodiment shown in FIG. 2, as shown in FIG. 7, the positioning method further includes steps S701-S703.

In step S701, whether the positioning state and/or the positioning deviation meet preset positioning requirement is determined. If the positioning state and/or the positioning deviation meet the preset positioning requirements, the process proceeds to the step S702. If the positioning state and/or the positioning deviation do not meet the preset positioning requirements, the process proceeds to the step S703.

In step S702, an identity authentication is performed on the target object, and the current positioning is ended when the identity authentication is passed.

In step S703, the positioning information is output, the current posture image of the target object is obtained and taken as a new second posture image, and the process returns to perform the step S202.

The preset positioning requirements may include a requirement that the positioning deviation is less than a preset deviation threshold, or a requirement that the positioning state is consistent with the preset positioning state, which can be determined in advance according to actual evaluation needs.

In the embodiments of the present disclosure, when the radiation delivery system obtains the positioning information based on the foregoing steps, it can further determine whether the positioning information meets the preset positioning requirements. If the positioning information meets the preset positioning requirements, it indicates that the deviation between the first posture and the second posture of the target object is small and the repeatability is strong, that is, it meets the positioning requirements. In this case, the radiation delivery system does not need to instruct the target object to adjust the posture, and may further perform an identity authentication on the target object by using a face or iris detection method. Here, face detection may be that the radiation delivery system identifies the identity information of the target object based on the face image of the target object, and iris detection may be that the radiation delivery system identifies the identity information of the target object based on the face image or posture image of the target object. When the identity information of the target object is determined, the legality or consistency of the target object can be authenticated according to the identity information, and if the identity authentication is passed, the current positioning is ended. Then, the radiation device in the radiation delivery system can be activated to perform a dose projection on the target area of the target object. If the positioning information does not meet the preset positioning requirements, the positioning information can be output, for example, in the form of voice or display image to guide the target object to correct the position. Then, when the target object adjusts the current posture according to the positioning information, the radiation delivery system can obtain the current posture image of the target object in real time, use the current posture image as a new second posture image, and return to perform the step S202. Essentially, the current posture of the target object is detected in real time, and then the foregoing steps are returned to re-determine the positioning information until the current posture of the target object can meet the preset positioning requirements.

The method described in the above embodiment continuously detects the current posture of the target object in real time during the positioning process, compares it with the initial posture of the target object, and automatically adjusts the posture of the target object in real time, providing a method for guiding the positioning of the target object through the radiation delivery system, and improving the positioning efficiency and accuracy.

In an exemplary embodiment, a method for outputting positioning information is provided. The method includes outputting the positioning information to the target object in the form of voice for guiding the positioning of the target object. The positioning information can be converted into voice positioning information, and the voice positioning information can be broadcasted by using a voice broadcaster in the radiation delivery system.

In the embodiments of the present disclosure, when the positioning device in the radiation delivery system obtains the positioning information based on the foregoing steps, since the positioning information is expressed in text or professional terms, in order to enable the target object to understand the voice broadcast, the positioning device can convert the positioning information into voice positioning information, that is, convert it into ordinary language expression, so as to clearly and efficiently guide the positioning of the target object.

Alternatively, another method for outputting positioning information is provided. The method includes outputting the positioning information to the target object in the form of an image for guiding the positioning of the target object. A positioning image is generated according to the positioning information, the first posture image, and the second posture image, and the positioning image is displayed to the target object by using a display in the radiation delivery system. The positioning image includes a positioning indication identifier, and the positioning indication identifier includes a standard indication identifier and an actual indication identifier. The standard indication identifier indicates the first posture of the target object in the first posture image (including a distance identifier and a direction identifier), and the actual indication identifier indicates the second posture of the target object in the second posture image (including a distance identifier and a direction identifier). This voice broadcast manner can allow the target object to clearly obtain the movement information and move according to the voice broadcast information, which can improve the positioning efficiency.

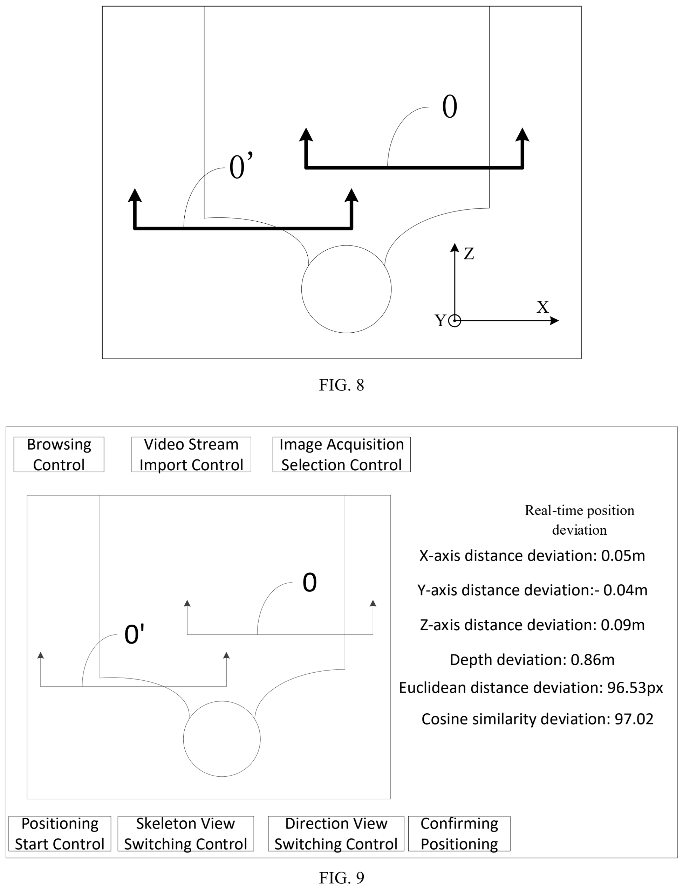

In the embodiments of the present disclosure, when the object positioning device in the radiation delivery system obtains the positioning information, the positioning deviation, the first posture image, and the second posture image based on the foregoing steps, it can identify the first posture in the first posture image to determine the first position of the target object, then identify the second posture in the second posture image to determine the second position of the target object. Since the second posture image is the current posture image of the target object, the object positioning device can generate a standard indication identifier and an actual indication identifier based on the positioning information, the position of the first position of the target object, and the position of the second position of the target object, and display the standard indication identifier and the actual indication identifier on the second posture image, or obtain the current posture image of the target object in real time and display the standard indication identifier and the actual indication identifier on the current posture image. For example, referring to the schematic diagram of the positioning image shown in FIG. 8, a standard indication identifier 0' and an actual indication identifier 0 are shown. The standard indication identifier 0' includes a position identifier and a direction identifier. The actual indication identifier 0 includes a position identifier and a direction identifier. It should be noted that the positioning image may include standard indication identifiers and actual indication identifiers corresponding to multiple different key points. Only one standard indication identifier and one actual indication identifier are shown in FIG. 8, which is only an example illustration, and the number of standard indication identifiers and actual indication identifiers is not limited. This image broadcast manner can allow the target object to more intuitively observe the deviation of its own position, and then adjust its own posture according to the guidance on the screen, which can improve the positioning efficiency.

In an exemplary embodiment, another object positioning method is provided, that is, on the basis of the embodiment shown in FIG. 2, the object positioning method further includes controlling a support platform in the radiation delivery system to drive the target object to move according to the instruction of the positioning information to complete positioning.

In the embodiments of the present disclosure, when the radiation delivery system obtains the positioning information based on the foregoing steps, it can guide the positioning of the target object based on the positioning information, generate an instruction according to the positioning information, and send the instruction to the controller of the support platform connected to the positioning device. After receiving the instruction, the controller can control the support platform to move according to the instruction to assist the target object in automatic positioning. This method can improve the positioning efficiency and accuracy by issuing instructions to the support platform to move the support platform for positioning the target object.

In an exemplary embodiment, a display interface of a display in the object positioning device is further provided, which can realize interaction with medical staff and operate the display of the positioning image. Referring to FIG. 9, the display interface includes an image display area, an information display area, and an interaction area. The image display area displays the positioning image, the information display area displays the positioning deviation, and the interaction area displays operation controls. The operation controls include at least one of an image acquisition selection control, a browsing control, a video stream import control, a positioning start control, a positioning indication identifier switching control, and a positioning saving control.

In the embodiments of the present disclosure, the display interface of the display is oriented to the target object. Therefore, referring to the schematic diagram shown in FIG. 1, the component of the object positioning device 102 closest to the support platform 103 is the display, so that when the target object is located on the support platform 103, the target object can intuitively see the display interface of the display 102 for positioning. It should be noted that, referring to the schematic diagram of the display interface shown in FIG. 9, the display interface displays a positioning image, which is obtained in real time and is an image with a positioning indication identifier added in real time. When the image acquisition selection control is triggered by the user, it is used to select a corresponding image acquisition device. For example, a plurality of cameras are arranged on the radiation delivery system. When the user triggers the image acquisition selection control, the corresponding camera can be selected to obtain images. When the browsing control is triggered by the user, it is used to obtain the second posture image of any fraction or the initial first posture image of the target object from the image database and display it in the image display area for the user to browse and view. When the video stream import control is triggered by the user, it is used to centrally obtain the first posture image or the second posture image of the target object from images obtained by other image acquisition devices, where the other image acquisition devices refer to image acquisition devices not on the radiation delivery system. For example, it can be a camera in the room where the radiation delivery system is located, and the camera can also obtain the first posture image or the second posture image of the target object. When the positioning start control is triggered by the user, it is used to display the positioning image in the image display area. When the positioning indication identifier switching control is triggered by the user, it is used to switch and display the positioning indication identifier on the positioning image. For example, only the standard indication identifier or the actual indication identifier is displayed on the positioning image, or both indication identifiers are displayed. Optionally, when the positioning indication identifier switching control is triggered by the user, it can also be used to switch and display or not display the indication identifier including the position deviation on the positioning image. For example, the skeleton view switching control in FIG. 9 refers to switching and displaying or not displaying the identifier including the position deviation, and it is also used to switch and display or not display the indication identifier including the orientation deviation on the positioning image. For example, the direction view switching control in FIG. 9 refers to switching and displaying or not displaying the indication identifier including the orientation deviation. When the positioning saving control is triggered by the user, it is used to save the current posture image of the target object as the fraction posture image of the current time.

The method described in the above embodiment provides an interactive interface, enabling medical staff to interact with the radiation delivery system during the automatic positioning of the target object according to the guidance of the radiation delivery system, assisting the target object in positioning, and enhancing the intelligence of the entire radiation delivery system. Moreover, in addition to the image for guiding positioning, the display interface also displays the positioning deviation information to the target object, enabling the target object to more intuitively and clearly understand the current posture, and the position and direction to move, thereby improving the positioning efficiency and accuracy.

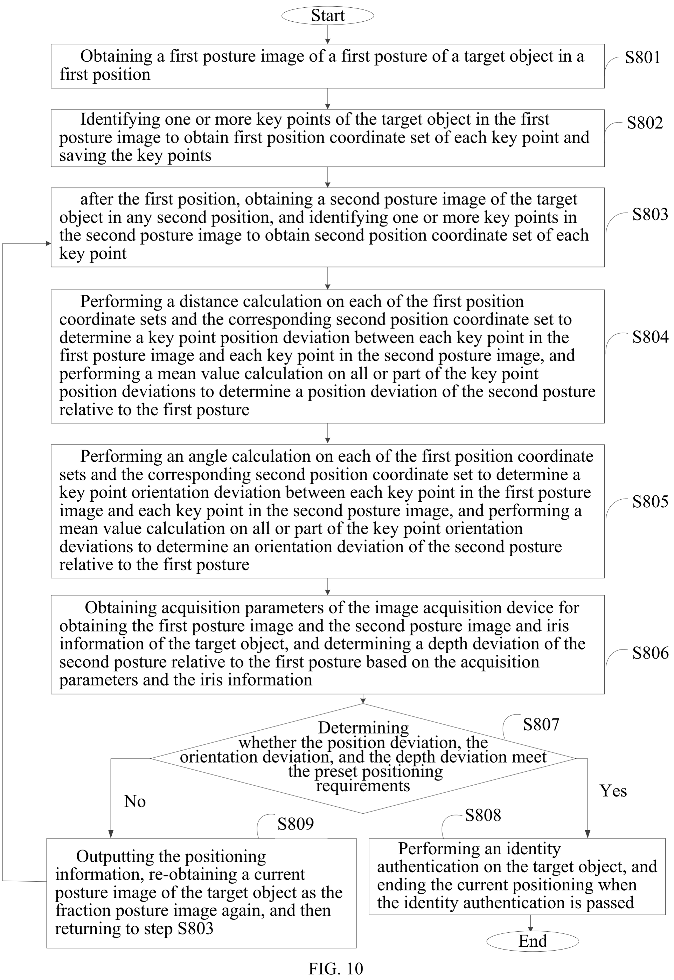

Combining the methods described in all the above embodiments, a positioning method is provided. As shown in FIG. 10, the method includes steps S801-S809.

In the step S801, a first posture image of a first posture of a target object in a first position is obtained.

In the step S802, one or more key points of the target object in the first posture image are identified to obtain a first position coordinate set of each key point, and the key points are saved.

In the step S803, after the first position, a second posture image of the target object in any second position is obtained, and one or more key points in the second posture image are identified to obtain a second position coordinate set of each key point.

In the step S804, a distance calculation is performed on each of the first position coordinate sets and the corresponding second position coordinate set to determine a key point position deviation between each key point in the first posture image and each key point in the second posture image, and a mean value calculation is performed on all or part of the key point position deviations to determine a position deviation of the second posture relative to the first posture.

In the step S805, an angle calculation is performed on each of the first position coordinate sets and the corresponding second position coordinate set to determine a key point orientation deviation between each key point in the first posture image and each key point in the second posture image, and a mean value calculation is performed on all or part of the key point orientation deviations to determine an orientation deviation of the second posture relative to the first posture.

In the step S806, acquisition parameters of the image acquisition device for obtaining the first posture image and the second posture image, and iris information of the target object are obtained, and a depth deviation of the second posture relative to the first posture is determined based on the acquisition parameters and the iris information.

In the step S807, whether the position deviation, the orientation deviation, and the depth deviation meet the preset positioning requirements is determined. If the preset positioning requirements are met, the method proceeds to step S808, and if the preset positioning requirements are not met, the method proceeds to step S809.

In the step S808, an identity authentication is performed on the target object, and the current positioning is ended when the identity authentication is passed.

In the step S809, the positioning information is output, the current posture image of the target object is re-obtained as the fraction posture image again, and then the method returns to step S803.

In combination with the foregoing description, in some embodiments, the present disclosure therefore also provides an object positioning method, including:

obtaining a posture image of a target object during a current treatment fraction;
determining current positions of one or more key points of the target object based on the posture image; and
determining positioning information of the target object by comparing the current positions with historical positions of the one or more key points.

In some embodiments, before obtaining the posture image of the target object during the current treatment fraction, the method further includes obtaining a historical posture image of the target object during a historical treatment fraction, and determining historical positions of one or more key points in the historical posture image as the historical positions, where the one or more key points in the posture image during the current treatment fraction are in one-to-one correspondence with the one or more key points in the historical posture image.

Other further features and beneficial effects of the object positioning method based on these embodiments can be obtained according to the foregoing description, and will not be repeated here.

Each of the above steps has been described in the foregoing, and the detailed content can be referred to the foregoing description, which will not be repeated here.

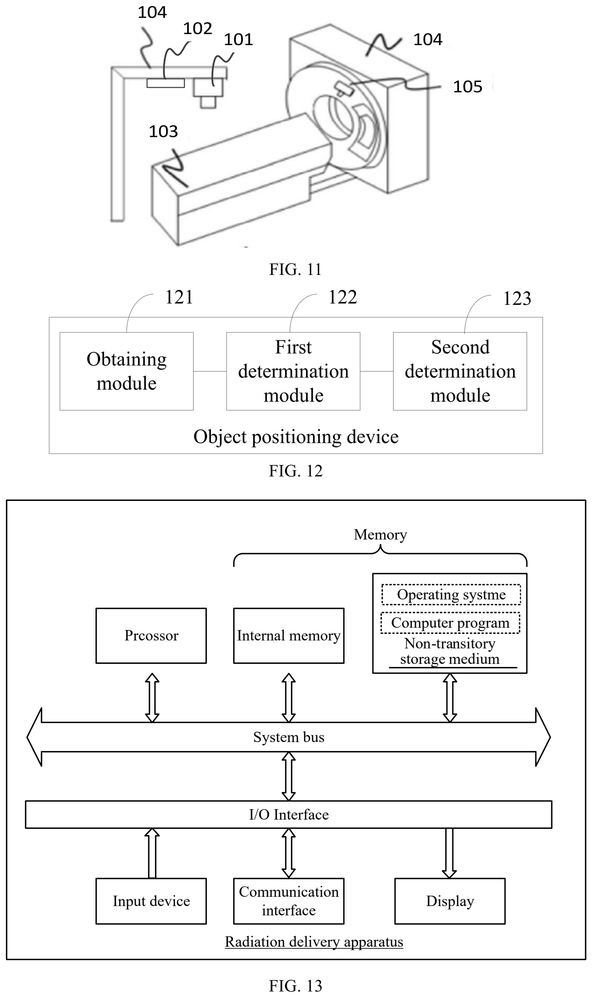

In an exemplary embodiment, based on the positioning methods described in all the above embodiments, a radiation delivery system is provided. The radiation delivery system can be used for radiation treatment, radiation verification with a phantom before radiotherapy, and the like. In the embodiments of the present disclosure, the radiation delivery system is used to guide the initial positioning of a target object during or before the formulation of a radiation delivery plan, and guide the delivery positioning of the target object after the formulation of the radiation delivery plan and during or before the radiation delivery. As shown in FIG. 11, the radiation delivery system includes an image acquisition device 101, an object positioning device 102, and a support platform 103. The image acquisition device 101 is configured to obtain a first posture image of the target object in the first position on the support platform 103 and a second posture image of the target object in any second position on the support platform 103. The object positioning device 102 is configured to perform key point recognition on the first posture image and the second posture image, determine a positioning deviation of the second posture relative to the first posture, and determine and output positioning information according to the positioning deviation.

The radiation delivery system described in the embodiments of the present disclosure is applied to the method described in any one of the foregoing embodiments in FIGS. 2-10. For the specific method implementation, reference can be made to the content described in the foregoing embodiments, which will not be repeated here.

In an exemplary embodiment, the object positioning device 102 shown in FIG. 11 further includes a processing component 1021, a display 1022, and/or a voice broadcaster 1023. The processing component 1021 is configured to perform key point recognition on the first posture image and the second posture image, determine the position coordinate sets of the first positions of the key points in the first posture image and the position coordinate sets of the second positions of the key points in the second posture image, thereby obtaining the positioning information of the second posture relative to the first posture. The voice broadcaster 1023 is configured to convert the positioning information into voice positioning information and broadcast the voice positioning information. The processing component 1021 is further configured to generate a positioning image according to the positioning information, the first posture image, and the second posture image. The positioning image includes a positioning indication identifier. The display 1022 is configured to display the positioning image to the target object.

The usage method of each component or module in the above radiation delivery system has been described in the foregoing, and the detailed content can be referred to the foregoing description, which will not be repeated here.

In an exemplary embodiment, the radiation delivery apparatus shown in FIG. 11 further includes a frame 104, the image acquisition device 101 is arranged on the frame 104, and the support platform 103 is located within the shooting range of the image acquisition device 101. The frame 104 may be a frame of an annular structure or a frame of a non-annular structure (the frame in the schematic diagram shown in FIG. 11). The frame 104 in FIG. 11 includes two parts of frames. One part is the frame connected to the support platform 103, which is an annular structure, and a treatment component 105 is arranged thereon. After the target object is positioned, the support platform 103 drives the target object to move into the ring of the frame, and the treatment component 105 is activated to perform dose projection on the target object. The other part is a frame located near the support platform 103, on which the image acquisition device 101 is arranged to obtain the posture image of the target object in real time. If the positioning device includes a display, the display 102 is also arranged on the frame, and its display screen faces the target object, so that the target object can perform positioning based on the instructions on the screen. If the positioning device includes a voice broadcaster, the voice broadcaster can also be arranged on the frame to broadcast the voice positioning information to the target object, so that the target object can clearly hear the voice broadcast at a short distance for positioning. It should be noted that the image acquisition device 101 can also be arranged on the frame of the annular structure in FIG. 11 to facilitate the acquisition of images of the target object on the support platform 103.

The usage method of each component or module in the above radiation delivery system has been described in the foregoing, and the detailed content can be referred to the foregoing description, which will not be repeated here.

It should be understood that although the various steps in the flowcharts involved in the above embodiments are displayed in sequence according to the arrows, these steps are not necessarily executed in sequence according to the order indicated by the arrows. Unless explicitly stated herein, the execution of these steps is not strictly limited in order, and these steps can be executed in other orders. Moreover, at least part of the steps in the flowcharts involved in the above embodiments may include multiple steps or multiple stages. These steps or stages are not necessarily executed at the same time, but can be executed at different times. The execution order of these steps or stages is not necessarily sequential, but can be rotated or alternately executed with at least part of the steps or stages in other steps or other stages.

Based on the same inventive concept, the embodiments of the present disclosure also provide an object positioning device for implementing the positioning method described above. The implementation solution for solving the problem provided by the device is similar to the implementation solution recorded in the above method, so the specific limitations in one or more object positioning device embodiments provided below can refer to the limitations on the positioning method above, and will not be repeated here.

In an exemplary embodiment, as shown in FIG. 12, an object positioning device is provided, including:

an obtaining module 121, configured to obtain a first posture image of a first posture of a target object in a first position and a second posture image of a second posture of the target object in a second position after the first position;

a first determination module 122, configured to determine first positions of one or more key points of the target object in the first posture image and second positions of the one or more key points in the second posture image; and

a second determination module 123, configured to determine positioning information of the target object based on the first positions and the second positions.

In an exemplary embodiment, the second determination module 123 is configured to determine a positioning state and/or a positioning deviation of the second posture relative to the first posture as the positioning information based on the first positions and the second positions.

In an exemplary embodiment, the first determination module 122 includes:

a first identification unit, configured to identify the one or more key points in the first posture image to obtain a first position coordinate set of each of the key points; and

a second identification unit, configured to identify the one or more key points in the second posture image to obtain a second position coordinate set of each of the key points, where the number and type of the key points in the first posture image are consistent with those of the key points in the second posture image, and each of the first position coordinate sets and each of the second position coordinate sets are used to determine the positioning information.

In an exemplary embodiment, the object positioning device further includes:

a first calculation module, configured to perform a distance calculation on each of the first position coordinate sets and the corresponding second position coordinate set to determine a key point position deviation between the key point corresponding to each of the first position coordinate sets and the key point corresponding to each of the second position coordinate sets;

a second calculation module, configured to perform an angle calculation on each of the first position coordinate sets and the corresponding second position coordinate set to determine a key point orientation deviation between the key point corresponding to each of the first position coordinate sets and the key point corresponding to each of the second position coordinate sets; and

a first position determination module, configured to determine the positioning information based on the key point position deviation and the key point orientation deviation.

In an exemplary embodiment, the positioning determination subunit is specifically configured to perform a mean value calculation on all or part of the key point position deviations to determine an average position deviation of the second posture relative to the first posture, and perform a mean value calculation on all or part of the key point orientation deviations to determine an average orientation deviation of the second posture relative to the first posture.

In an exemplary embodiment, the object positioning device further includes:

an information obtaining module, configured to obtain acquisition parameters for acquiring the second posture image and iris information of the target object. The acquisition parameters include a field of view angle parameter and an image width. The iris information includes the actual size of the iris of the target object and the pixel size of the iris; and

a second position determination module, configured to determine the depth deviation of the second posture relative to the first posture based on the acquisition parameters and the iris information.

Each module in the above object positioning device can be fully or partially implemented by software, hardware, or a combination thereof. Each of the above modules can be embedded in or independent of the processor in the computer device in the form of hardware, or stored in the memory in the computer device in the form of software, so that the processor can call and execute the corresponding operations of each of the above modules.

In an exemplary embodiment, a radiation delivery apparatus is provided. The radiation delivery apparatus may be a terminal, and its internal structure diagram may be as shown in FIG. 13. The radiation delivery apparatus includes a processor, a memory, an input/output interface, a communication interface, a display unit, and an input device. The processor, the memory, and the input/output interface are connected through a system bus, and the communication interface, the display unit, and the input device are connected to the system bus through the input/output interface. The processor of the radiation delivery apparatus is used to provide computing and control capabilities. The memory of the radiation delivery apparatus includes a non-volatile storage medium and an internal memory. The non-volatile storage medium stores an operating system and a computer program. The internal memory provides an environment for the operation of the operating system and the computer program in the non-volatile storage medium. The input/output interface of the radiation delivery apparatus is used to exchange information between the processor and external devices. The communication interface of the radiation delivery apparatus is used to communicate with an external terminal in a wired or wireless manner, and the wireless manner can be implemented through WIFI, a mobile cellular network, Near Field Communication (NFC), or other technologies. The computer program is executed by the processor to implement an object positioning method. The display unit of the radiation delivery apparatus is used to form a visually visible picture, and may be a display screen, a projection device, or a virtual reality imaging device. The display screen may be a liquid crystal display screen or an electronic ink display screen. The input device of the radiation delivery apparatus may be a touch layer covered on the display screen, or a button, a trackball, or a touchpad provided on the housing of the radiation delivery apparatus, or an external keyboard, touchpad, or mouse, etc.

Those skilled in the art can understand that the structure shown in FIG. 13 is only a block diagram of a part of the structure related to the solution of the present disclosure, and does not constitute a limitation on the radiation delivery apparatus to which the solution of the present disclosure is applied. A specific radiation delivery apparatus may include more or fewer components than those shown in the figure, or combine some components, or have different component arrangements.

In an exemplary embodiment, a computer device is provided, including a memory and a processor. The memory stores a computer program, and the processor, when executing the computer program, is configured to perform the following steps:

obtaining a first posture image of a first posture of a target object in a first position and a second posture image of a second posture of the target object in a second position after the first position;
determining first positions of one or more key points of the target object in the first posture image and second positions of the one or more key points in the second posture image; and
determining positioning information of the target object based on the first positions and the second positions.

The implementation principle and technical effect of the computer device provided in the above embodiment are similar to those of the above method embodiment, and will not be repeated here.

In an embodiment, a non-transitory computer-readable storage medium is provided, on which a computer program is stored. The computer program, when executed by a processor, causes the processor to perform the following steps:

obtaining a first posture image of a first posture of a target object in a first position and a second posture image of a second posture of the target object in a second position after the first position;
determining first positions of one or more key points of the target object in the first posture image and second positions of the one or more key points in the second posture image; and
determining positioning information of the target object based on the first positions and the second positions.

The implementation principle and technical effect of the computer-readable storage medium provided in the above embodiment are similar to those of the above method embodiment, and will not be repeated here.

In an embodiment, a computer program product is provided, including a computer program, which is executed by a processor to implement the following steps:

obtaining a first posture image of a first posture of a target object in a first position and a second posture image of a second posture of the target object in a second position after the first position;
determining first positions of one or more key points of the target object in the first posture image and second positions of the one or more key points in the second posture image; and
determining positioning information of the target object based on the first positions and the second positions.

The implementation principle and technical effect of the computer program product provided in the above embodiment are similar to those of the above method embodiment, and will not be repeated here.

Those of ordinary skill in the art can understand that all or part of the processes in the methods of the above embodiments can be implemented by instructing relevant hardware through a computer program. The computer program can be stored in a non-volatile computer-readable storage medium. When the computer program is executed, it can include the processes of the above method embodiments. Any reference to a memory, a database, or other media in the embodiments provided in the present disclosure may include at least one of a non-volatile memory and a volatile memory. The non-volatile memory may include a Read-Only Memory (ROM), a magnetic tape, a floppy disk, a flash memory, an optical memory, a high-density embedded non-volatile memory, a Resistive Random Access Memory (ReRAM), a Magnetoresistive Random Access Memory (MRAM), a Ferroelectric Random Access Memory (FRAM), a Phase Change Memory (PCM), a graphene memory, etc. The volatile memory may include a Random Access Memory (RAM) or an external cache memory, etc. By way of illustration and not limitation, the RAM may be in various forms, such as a Static Random Access Memory (SRAM) or a Dynamic Random Access Memory (DRAM). The databases involved in the embodiments provided in the present disclosure may include at least one of a relational database and a non-relational database. The non-relational database may include a distributed database based on a blockchain, etc., without limitation. The processors involved in the embodiments provided in the present disclosure may be general-purpose processors, central processing units, graphics processors, digital signal processors, programmable logic devices, data processing logic devices based on quantum computing, artificial intelligence (AI) processors, etc., without limitation.

The technical features of the above embodiments can be combined arbitrarily. To make the description concise, not all possible combinations of the technical features in the above embodiments are described. However, as long as there is no contradiction between the combinations of these technical features, they should be regarded as the scope of the present disclosure.

The above-described embodiments only express several implementation modes of the present disclosure, and the description thereof is relatively specific and detailed, but it should not be understood as a limitation on the patent scope of the present disclosure. It should be pointed out that for those of ordinary skill in the art, without departing from the concept of the present disclosure, several modifications and improvements can be made, which all fall within the protection scope of the present disclosure. Therefore, the protection scope of the present disclosure should be subject to the appended claims.

An object positioning method includes obtaining a first posture image of a first posture of a target object in a first position, and determining first positions of one or more key points of the target object in the first posture image; obtaining a second posture image of a second posture of the target object in a second position after the first position, and determining second positions of the one or more key points in the second posture image; and determining positioning information of the target object based on the first positions and the second positions.

**Claims**

1. An object positioning method, comprising:

   obtaining a first posture image of a first posture of a target object in a first position, and determining first positions of one or more key points of the target object in the first posture image;
   obtaining a second posture image of a second posture of the target object in a second position after the first position, and determining second positions of one or more key points of the target object in the second posture image; and
   determining positioning information of the target object based on the first positions and the second positions.

2. The object positioning method according to claim 1, wherein the positioning information comprises a positioning state and/or a positioning deviation of the second posture relative to the first posture.

3. The object positioning method according to claim 2, further comprising:
   determining whether the positioning state and/or the positioning deviation meet preset positioning requirements.

4. The object positioning method according to claim 3, further comprising:
   performing an identity authentication on the target object if the positioning state and/or the positioning deviation meet the preset positioning requirements.

5. The object positioning method according to claim 3, further comprising:
   obtaining a current posture image of the target object if the positioning state and/or the positioning deviation do not meet the preset positioning requirements, taking the current posture image as a new second posture image, determining second positions of one or more key points in the new second posture image, and determining the positioning information of the target object based on the first positions and second positions of the one or more key points in the new second posture image.

6. The object positioning method according to claim 1, comprising:
outputting the positioning information to the target object in a form of voice and/or image for guiding positioning of the target object.

7. The object positioning method according to claim 1, wherein
determining the first positions of the one or more key points of the target object in the first posture image comprises:

identifying the one or more key points in the first posture image to obtain first position coordinate sets of the one or more key points in the first posture image;
determining the second positions of the one or more key points of the target object in the second posture image comprises:

identifying the one or more key points in the second posture image to obtain second position coordinate sets of the one or more key points in the second posture image;
the one or more key points in the first posture image are in one-to-one correspondence with the one or more key points in the second posture image; and
determining the positioning information of the target object based on the first positions and the second positions comprises:
determining the positioning information based on the first position coordinate sets and the second position coordinate sets.

8. The object positioning method according to claim 7, wherein the positioning information comprises a position deviation and an orientation deviation, and determining the positioning information based on the first position coordinate sets and the second position coordinate sets comprises:

performing a distance calculation on the first position coordinate sets and the corresponding second position coordinate sets to determine key point position deviations between the key points corresponding to the first position coordinate sets and the key points corresponding to the second position coordinate sets;
performing an angle calculation on the first position coordinate sets and the corresponding second position coordinate set to determine key point orientation deviations between the key points corresponding to the first position coordinate sets and the key points corresponding to the second position coordinate sets; and
determining the positioning information based on the key point position deviations and the key point orientation deviations.

9. The object positioning method according to claim 8, wherein determining the positioning information based on the key point position deviations and the key point orientation deviations comprises:

performing a mean value calculation on all or part of the key point position deviations to determine an average position deviation of the second posture relative to the first posture; and
performing a mean value calculation on all or part of the key point orientation deviations to determine an average orientation deviation of the second posture relative to the first posture.

10. The object positioning method according to claim 1, wherein the positioning information comprises a depth deviation, and the object positioning method further comprises:

obtaining acquisition parameters for acquiring the second posture image and iris information of the target object; and
determining the depth deviation of the second posture relative to the first posture based on the acquisition parameters and the iris information.

11. The object positioning method according to claim 1, wherein the first position is an initial position of the target object during or before formulation of a current radiation delivery plan, and the second position is a delivery position of the target object after the formulation of the current radiation delivery plan and during or before the current radiation delivery.

12. The object positioning method according to claim 1, comprising:
moving the target object based on the positioning information to complete positioning of the target object.

13. A radiation delivery system, comprising an image, a positioning device, and a support platform,

wherein the image acquisition device is configured to obtain a first posture image of a first posture of a target object in a first position on the support platform and a second posture image of a second posture of the target object in a second position on the support platform after the first position; and
the positioning device is configured to determine first positions of one or more key points of the target object in the first posture image and second positions of the one or more key points in the second posture image, and determine positioning information based on the first positions and the second positions.

14. The radiation delivery system according to claim 13, wherein the support platform is configured to move the target object based on the positioning information to complete positioning of the target object.

15. The radiation delivery system according to claim 13, wherein determining the first positions of the one or more key points of the target object in the first posture image and the second positions of the one or more key points in the second posture image comprises:

identifying the one or more key points in the first posture image to obtain first position coordinate sets of the one or more key points of the target object in the first posture image; and
identifying the one or more key points in the second posture image to obtain second position coordinate sets of the one or more key points of the target object in the second posture image;
wherein the one or more key points in the first posture image are in one-to-one correspondence with the one or more key points in the second posture image; and
determining the positioning information based on the first positions and the second positions comprises:
determining the positioning information based on the first position coordinate sets and the second position coordinate sets.

102

101 102

103

FIG. 1

obtaining a first posture image of a first posture of a target object in a first position, and determining first positions of one or more key points of the target object in the first posture image

S201

obtaining a second posture image of a second posture of the target object in a second position after the first position, and determining second positions of one or more key points of the target object in the second posture image

S202

determining positioning information of the target object based on the first positions and the second positions

S203

FIG. 2

identifying the one or more key points in the first posture image to obtain first position coordinate sets of the one or more key points in the first posture image

S301

identifying the one or more key points in the second posture image to obtain second position coordinate sets of the one or more key points in the second posture image

S302

FIG. 3

performing a distance calculation on the first position coordinate sets and the corresponding second position coordinate set to determine key point position deviations between the key points corresponding to the first position coordinate sets and the key points corresponding to the second position coordinate sets

S401

performing an angle calculation on the first position coordinate sets and the corresponding second position coordinate set to determine key point orientation deviations between the key points corresponding to the first position coordinate sets and the key points corresponding to the second position coordinate sets

S402

determining the positioning information based on the key point position deviations and the key point orientation deviations

S403

FIG. 4

performing a mean value calculation on all or part of the key point position deviations to determine an average position deviation of the second posture relative to the first posture — S501

performing a mean value calculation on all or part of the key point orientation deviations to determine an average orientation deviation of the second posture relative to the first posture — S502

FIG. 5

obtaining acquisition parameters for acquiring the second posture image and iris information of the target object — S601

determining the depth deviation of the second posture relative to the first posture based on the acquisition parameters and the iris information — S602

FIG. 6

determining whether the positioning state and/or the positioning deviation meet preset positioning requirements — S701

No

Yes

Outputing the positioning information, obataining the current posture image of the target object as a new second posture image, and returning to perform the step S202. — S703

performing an identity authentication on the target object, and ending the current positioning when the identity authentication is passed — S702

FIG. 7

FIG. 8

| | | |
|---|---|---|
| Browsing Control | Video Stream Import Control | Image Acquisition Selection Control |

Real-time position deviation

X-axis distance deviation: 0.05m

Y-axis distance deviation:- 0.04m

Z-axis distance deviation: 0.09m

Depth deviation: 0.86m

Euclidean distance deviation: 96.53px

Cosine similarity deviation: 97.02

| | | | |
|---|---|---|---|
| Positioning Start Control | Skeleton View Switching Control | Direction View Switching Control | Confirming Positioning |

FIG. 9

Start

Obtaining a first posture image of a first posture of a target object in a first position — S801

Identifying one or more key points of the target object in the first posture image to obtain first position coordinate set of each key point and saving the key points — S802

after the first position, obtaining a second posture image of the target object in any second position, and identifying one or more key points in the second posture image to obtain second position coordinate set of each key point — S803

Performing a distance calculation on each of the first position coordinate sets and the corresponding second position coordinate set to determine a key point position deviation between each key point in the first posture image and each key point in the second posture image, and performing a mean value calculation on all or part of the key point position deviations to determine a position deviation of the second posture relative to the first posture — S804

Performing an angle calculation on each of the first position coordinate sets and the corresponding second position coordinate set to determine a key point orientation deviation between each key point in the first posture image and each key point in the second posture image, and performing a mean value calculation on all or part of the key point orientation deviations to determine an orientation deviation of the second posture relative to the first posture — S805

Obtaining acquisition parameters of the image acquisition device for obtaining the first posture image and the second posture image and iris information of the target object, and determining a depth deviation of the second posture relative to the first posture based on the acquisition parameters and the iris information — S806

Determining whether the position deviation, the orientation deviation, and the depth deviation meet the preset positioning requirements — S807

No

S809

Outputting the positioning information, re-obtaining a current posture image of the target object as the fraction posture image again, and then returning to step S803

Yes

S808

Performing an identity authentication on the target object, and ending the current positioning when the identity authentication is passed

End

FIG. 10

FIG. 11

| Obtaining module | First determination module | Second determination module |
|---|---|---|
| 121 | 122 | 123 |

Object positioning device

FIG. 12

FIG. 13

## EUROPEAN SEARCH REPORT

**Application Number**

EP 25 22 5128

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/203123 A1 (VOJAN FILIP [US] ET AL) 30 June 2022 (2022-06-30)<br>* the whole document *<br>* figure 40 *<br>* paragraphs [0227] - [0231] *<br>* paragraphs [0312], [0347] *<br>----- | 1-15 | INV.<br>A61N5/10 |
| X | US 2018/353774 A1 (MEIR IVAN DANIEL [GB] ET AL) 13 December 2018 (2018-12-13)<br>* the whole document *<br>* paragraph [0002] *<br>* paragraphs [0025] - [0028] *<br>* paragraph [0049] *<br>----- | 1-3,5,6,<br>11-14 | |
| X | US 2016/067525 A1 (BOUCHET LIONEL G [US] ET AL) 10 March 2016 (2016-03-10)<br><br>* the whole document *<br>* paragraph [0057] *<br>* paragraphs [0068] - [0075] *<br>* claim 19 *<br>----- | 1,2,5,<br>7-9,<br>11-15 | |
| X | US 2019/366124 A1 (BERLINGER KAJETAN [DE]) 5 December 2019 (2019-12-05)<br>* the whole document *<br>* figure 1 *<br>* paragraphs [0032] - [0038] *<br>* paragraphs [0046] - [0048] *<br>* paragraphs [0065], [0093] *<br>----- | 1-3,5-9,<br>11-15 | **TECHNICAL FIELDS SEARCHED (IPC)**<br><br>A61N |

The present search report has been drawn up for all claims

| Place of search | Date of completion of the search | Examiner |
|---|---|---|
| Munich | 9 April 2026 | Seiferle, Benedict |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 22 5128

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2026

| Patent document cited in search report | | Publication date | Patent family member(s) | | Publication date |
|---|---|---|---|---|---|
| US 2022203123 | A1 | 30-06-2022 | US | 12268896 B1 | 08-04-2025 |
| | | | US | 2022203123 A1 | 30-06-2022 |
| US 2018353774 | A1 | 13-12-2018 | CN | 108992796 A | 14-12-2018 |
| | | | EP | 3412341 A1 | 12-12-2018 |
| | | | GB | 2563256 A | 12-12-2018 |
| | | | JP | 7156824 B2 | 19-10-2022 |
| | | | JP | 2018202155 A | 27-12-2018 |
| | | | US | 2018353774 A1 | 13-12-2018 |
| US 2016067525 | A1 | 10-03-2016 | CN | 105561484 A | 11-05-2016 |
| | | | EP | 2995348 A1 | 16-03-2016 |
| | | | ES | 2716130 T3 | 10-06-2019 |
| | | | JP | 6643830 B2 | 12-02-2020 |
| | | | JP | 2016055161 A | 21-04-2016 |
| | | | US | 2016067525 A1 | 10-03-2016 |
| US 2019366124 | A1 | 05-12-2019 | EP | 3461296 A1 | 03-04-2019 |
| | | | EP | 3586309 A1 | 01-01-2020 |
| | | | US | 2019156478 A1 | 23-05-2019 |
| | | | US | 2019156497 A1 | 23-05-2019 |
| | | | US | 2019366124 A1 | 05-12-2019 |
| | | | WO | 2018153473 A1 | 30-08-2018 |
| | | | WO | 2018153675 A1 | 30-08-2018 |

EPO FORM P0459